# EUROPÄISCHE PATENTSCHRIFT

(11) **EP 1 440 694 B1**
(45) Veröffentlichungstag und Bekanntmachung des Hinweises auf die Patenterteilung: **21.02.2007**
(21) Anmeldenummer: 03029478.9
(22) Anmeldetag: 19.12.2003
(51) Int. Cl.: A61L 2/26, B67C 7/00, B67C 3/26

(54) **Sterilisiervorrichtung**
Sterilization device
Dispositif de stérilization

(30) Priorität: 14.01.2003 DE 20300522 U
(43) Veröffentlichungstag der Anmeldung: 28.07.2004
(73) Patentinhaber: Salda, Luciano, 41058 Vignola (MO) (IT)
(72) Erfinder: Salda, Luciano, 41058 Vignola (MO) (IT)
(74) Vertreter: Grünecker, Kinkeldey, Stockmair & Schwanhäusser Anwaltssozietät

(56) Entgegenhaltungen:
- WO-A-01/14241
- DE-A- 3 809 855
- FR-A- 2 485 478
- US-A- 3 486 840
- US-B1- 6 230 472

## Beschreibung

Die vorliegende Erfindung betrifft eine Sterilisiervorrichtung gemäß dem Oberbegriff des Anspruches 1. Sie kann beispielsweise im medizinischen Bereich oder im Lebensmittelbereich eingesetzt werden, um Behälter zu sterilisieren, insbesondere Flaschen.

Eine Sterilisiervorrichtung ist aus der DE 198 08 318 A1 bekannt. Bei dieser bekannten Sterilisiervorrichtung durchlaufen die zu sterilisierenden Behälter nacheinander drei Rundläufer. Auf dem ersten Rundläufer werden sie sterilisiert, auf dem zweiten erfolgt das Ausspülen des Sterilisiergemisches, und auf dem dritten Rundläufer werden die Behälter befüllt. Alle drei Rundläufer sind in einem gemeinsamen Raum untergebracht, der die Sterilkammer für die gesamte Vorrichtung darstellt. Diese sehr große Sterilkammer wird durch einen laminaren Sterilluftstrom steril gehalten, der oben in den Raum eingeleitet wird und mit einer Geschwindigkeit von zirka 0,5 m pro Sekunde nach unten sinkt. Für diese Sterilisierungstechnik wird eine außerordentlich große Menge an Sterilluft benötigt, was einen hohen technischen Aufwand und dementsprechend hohe Kosten bedeutet.

Die WO 01/142 41 beschreibt eine Maschine zum Befüllen von Behältern. Ein Sterilisieren der Behälter wird nirgendwo erwähnt. Stattdessen besteht das Anliegen der WO 01/142 41 darin, den atmosphärischen Druck um die Behälteröffnung herum während des Befüllens zu kontrollieren.

Eine Vorrichtung zum sterilen Abfüllen von Flüssigkeiten im Behälter ist aus der DE 3 809 855 A1 bekannt. Bei der dort beschriebenen Vorrichtung wird der zu befüllende Behälter in eine einteilige Sterilglocke eingeführt, bevor er durch Dampf sterilisiert wird.

Aufgabe der vorliegenden Erfindung ist es, eine bekannte Sterilisiervorrichtung dahingehend zu verbessern, dass der technische Aufwand und die Kosten für die Sterilisierung geringer werden, während gleichzeitig die Qualität der Sterilisierung gewahrt bleibt.

Diese Aufgabe wird gelöst durch eine Sterilisiervorrichtung mit den Merkmalen des Anspruches 1.

Bei der vorliegenden Erfindung wird die Sterilkammer innerhalb der lokal begrenzten Sterilglocke gebildet. Durch das Sterilisiergas wird innerhalb der Sterilglocke eine lokale sterile Atmosphäre geschaffen, in der der Behälter nach dem Sterilisieren befüllt werden kann. Die Sterilglocke, die den Füllkopf und die Befüllöffnung des Behälters umgibt, bietet den enormen Vorteil, dass nun nicht mehr der gesamte, die Sterilisiervorrichtung enthaltende Raum sterilisiert werden muss. Statt dessen wird die Sterilkammer auf den kleinstmöglichen Raum reduziert, namentlich den Bereich zwischen dem Füllkopf der Füllvorrichtung und der Befüllöffnung des Behälter.

Das Ergebnis ist eine drastische Reduzierung der benötigten Menge an Sterilisiergas, wodurch auch die zur Erzeugung oder Speicherung des Sterilisiergases benötigten Anlagen kleiner dimensioniert werden können. Mit der erfindungsgemäßen Sterilisiervorrichtung wird nicht nur gegenüber herkömmlichen Anlagen Platz gespart, sondem die Füllmaschinen-Sterilisierung und die Abfüllung lassen sich zudem deutlich kostengünstiger durchführen.

Gemäß der Erfindung weist die Sterilglocke zudem wenigstens zwei Wandelemente auf. Sie können jeweils verschiedene Funktionen erfüllen und sich zum Bilden der Sterilkammer mittels des Schließelementes zusammenführen lassen. Dies bietet den Vorteil, dass der zwischen den Wandelementen umfasste Raum in der Bereitschafts-Stellung groß ist und so das Einführen der Befüllöffnung des Behälters erleichtert, während der umfasste Raum in der Sterilstellung minimiert wird.

Zweckmäßigerweise wird die Sterilglocke vor Produktionsbeginn in ihre Sterilstellung gebracht, wobei ein als Flasche geformter Behälter (dummy) die Sterilglocke abschließt. In dieser Sterilstellung wird die Sterilglocke selbst durch Einleiten des Sterilisiergases sterilisiert.

Zur Aufrechterhaltung des sterilen Zustandes der Sterilglocke ist es günstig, wenn permanent ein steriles Gas in diese geleitet wird. Dies gilt insbesondere, wenn in Sterilstellung der Glocke ein Behälter mit einem Produkt befüllt wird.

Es ist vorteilhaft, wenn die Sterilglocke in ihrer Sterilstellung die Befüllöffnung des Behälters zumindest weitgehend schließend umgibt, so dass nur ein geringer Anteil des sterilen Gases nach außen dringt und innerhalb der Glocke ein leichter Überdruck erzeugt wird, der das Eindringen von unsteriler Umgebungsluft verhindert.

In ihrer einfachsten Ausführungsform ist die Sterilglocke einteilig. Sie kann dann zum Beispiel über einen die Befüllöffnung umfassenden Abschnitt eines Behälters übergestülpt werden und mit der Außenseite des Behälters die Sterilkammer abdichten.

In der einfachsten Ausführungsform einer mehrteiligen Sterilglocke weist die Sterilglocke zwei Wandelemente auf, wobei mindestens eines der beiden Wandelemente relativ zu dem anderen bewegbar ist. Diese Sterilglocke kann so ausgeführt sein, dass sie sich an unterschiedlich geformte Behälter anpasst, beispielsweise an verschiedene Behältergrößen.

In einer speziellen Variante sind die beiden Wandelemente auf die Bereitschafts-Stellung der Sterilglocke hin gegeneinander vorgespannt. Dies bietet den Vorteil, dass sich die Sterilglocke bei dem Fehlen äußerer Kräfte von allein in ihre Bereitschafts-Stellung bewegt, in der die Befüllöffnung eines Behälters aufgenommen werden kann. Dies erleichtert den Betrieb der erfindungsgemäßen Sterilisiervorrichtung, da die Aufnahme der Befüllöffnung des Behälters in der Sterilglocke erleichtert wird.

Um die zum Bewegen der Wandelemente aufzuwendende Kraft zu verringern, kann mit jedem bewegbaren Wandelement ein Hebelkörper verbunden sein, der durch ein Hebelelement beaufschlagbar ist. Die aufzuwendende Kraft verringert sich durch die Hebelwirkung dieser Elemente.

Das Hebelelement selbst kann beispielsweise gabelförmig ausgeführt sein. Jeder der Gabelarme kann zum Bewegen eines der Wandelemente dienen, und zwischen den Gabelarmen kann die Sterilglocke aufgenommen sein.

Die aufzuwendende Kraft kann noch weiter verringert werden, wenn an dem Hebelkörper ein Reibungsminderer vorgesehen ist. Dieser kann entweder eine Oberfläche mit einer besonders geringen Gleitreibung aufweisen, oder beispielsweise als Rolle ausgebildet sein, damit der Reibungsminderer am Hebelelement abgleiten oder abrollen kann.

Zweckmäßig ist es, die Sterilglocke an der Füllvorrichtung anzuordnen. Auf diese Weise hat die Sterilglocke stets eine definierte Position gegenüber dem Füllkopf der Füllvorrichtung.

Eine Möglichkeit zum Abdichten der Sterilglocke zumindest in ihrer Sterilstellung bietet sich durch die Füllvorrichtung, insbesondere durch deren Füllkopf. Dazu ist es zweckmäßig die Form der Sterilglocke an die Form des Füllkopfes anzupassen. Gegebenenfalls kann am Füllkopf zudem ein Abdichtelement vorgesehen sein.

Bevorzugt ist der Füllkopf relativ zur Sterilglocke in deren Sterilstellung bewegbar. Dadurch lassen sich das Sterilisieren und das Befüllen nacheinander besonders gut durchführen. Während des Sterilisierens verharrt der Füllkopf in einer Wartestellung, wobei die Befüllöffnung des Behälters frei liegt und Sterilisiergas in den Behälter eindringen kann. Anschließend verfährt der Füllkopf relativ zur Sterilglocke und legt sich an die Befüllöffnung an oder wird sogar abschnittsweise in die Befüllöffnung eingeführt, um zielgerichteter befüllen zu können.

Um den Füllkopf in der Sterilglocke zu bewegen, kann auch die Sterilglocke in ihrer Sterilstellung relativ zur Füllvorrichtung veränderbar sein. Dafür sollte eine Führung vorgesehen sein, entlang derer die Sterilglocke bewegbar ist, damit sie eine definierte Bewegungsbahn beibehält.

Um die Bewegungsbahn besonders einfach zu gestalten, kann die Führung geradlinig sein. Dies bietet zudem die Möglichkeit, dass die mittels des Hebelelementes auf einen Hebelkörper ausgeübte Kraft etwa parallel zu der Führung gerichtet ist. Das Hebelelement bewirkt auf diese Weise nicht nur eine Bewegung der Wandelemente der Sterilglocke relativ zueinander, sondern kann zudem eine Bewegung der gesamten Sterilglocke entlang der Führung erlauben.

Vorzugsweise ist die Sterilglocke gegenüber der Füllvorrichtung federnd gelagert. Mittels der federnden Lagerung kann eine bestimmte Andrückkraft der Sterilglocke an den zu sterilisierenden Behälter erzielt werden, damit die Sterilglocke möglichst dicht an dem Behälter anliegt.

Sehr vorteilhaft ist es, wenn mindestens ein Positionierelement zum Positionieren eines Behälters gegenüber der Füllvorrichtung vorgesehen ist. Insbesondere kann der Behälter so positioniert werden, dass der Füllkopf der Füllvorrichtung günstig im Bezug auf die Befüllvorrichtung des Behälters ausgerichtet ist. Das Positionierelement sorgt zudem dafür, dass der Behälter diese vorteilhafte Position beibehält.

Das Positionierelement kann zum Beispiel ein Greifer sein, mittels dessen ein Behälter ergriffen werden kann. Ist die Form der zu sterilisierenden Behälter bekannt, so kann der Griff an diese Form angepasst sein, beispielsweise an einen bestimmten Abschnitt der zu sterilisierenden Behälter.

In einer Variante der erfindungsgemäßen Sterilisiervorrichtung ist das Positionierelement durch die Sterilglocke in deren Sterilstellung beaufschlagbar. Die Sterilglocke kann nun gegenüber dem Positionierelement abgedichtet werden, was den Vorteil bietet, dass diese Abdichtung unabhängig von der Form des zu sterilisierenden Behälters ist.

Besonders günstig ist es, wenn das Positionierelement selbst an der Sterilglocke angeordnet ist. Damit wird die Lage der Befüllöffnung des Behälters in der Sterilglocke eindeutig festgelegt.

Beispielsweise kann das Positionierelement an der Sterilglocke als Aussparung zur Aufnahme eines Behälterabschnittes ausgebildet sein. Bewegbare Wandelemente der Sterilglocke können dabei als Greifer für die Behälter dienen.

Es sind verschiedene Varianten denkbar, wie der Füllkopf und die Befüllöffnung des Behälters zueinander gebracht werden können. Denkbar wäre es, dass das Positionierelement relativ zur Füllvorrichtung verfahrbar ist. Dies hätte den Vorteil, dass das mechanisch aufwendige Bewegen der Füllvorrichtung vermieden wird.

Denkbar wäre es auch, dass lediglich der Füllkopf relativ zum Positionierelement verfahrbar ist. Auch dabei wird das Verfahren der gesamten Füllvorrichtung vermieden, und die Bewegung wird auf den Füllkopf beschränkt.

Besonders günstig ist es, wenn jedem Positionierelement genau eine Sterilglocke zugeordnet ist, da dann das Einführen eines Behälters in die Sterilglocke mittels des Positionierelementes besonders definiert vorgenommen werden kann.

Günstig ist es auch, wenn jedem Positionierelement und/oder jeder Sterilglocke genau ein Füllkopf zugeordnet ist. Der Füllkopf kann auf diese Weise so ausgerichtet werden, dass er besonders gut gegenüber der Befüllöffnung des Behälters positioniert ist.

In einer vorteilhaften Ausführungsform sind das Positionierelement und das Schließelement identisch, in dem der zu sterilisierende Behälter mittels des Positionierelementes in eine Sterilstellung verfahrbar ist, in der die Sterilglocke die Befüllöffnung des Behälters umgibt. Das Positionierelement wirkt folglich als Schließelement, und es entfällt die Notwendigkeit, zusätzlich zum Positionierelement noch ein Schließelement vorsehen zu müssen.

Um die Bewegung des Schließelementes zu steuern, ist eine Kurvensteuerung besonders geeignet. Sie kann vorgeben, wann das Schließelement die Sterilglocke von deren Bereitschafts-Stellung in die Schließstellung zu überführen hat.

Diese Kurvensteuerung zum Steuern des Schließelementes kann an einer Transportvorrichtung vorgesehen sein, entlang derer die zu sterilisierenden Behälter transportiert werden. An der Transportvorrichtung können zudem die Positionierelemente befestigt sein. Sie positionieren den Behälter, während dieser an der Transportvorrichtung entlang läuft und nacheinander sterilisiert und befüllt wird.

Im Folgenden werden drei Ausführungsbeispiele von Sterilisiervorrichtungen anhand einer Zeichnung beschrieben. Dabei sind die Ausführungsbeispiele der Figuren 1 bis 4 und 9 nicht Teil der Erfindung. Im Einzelnen zeigen:
- Figur 1: einen Vertikalschnitt durch ein Ausführungsbeispiel einer nicht erfindungsgemäßen Sterilisiervorrichtungmit einer einteiligen Sterilglocke, wobei sich die Sterilglocke in ihrer Bereitschaftsstellung befindet,
- Figur 2: einen Vertikalschnitt durch das in Figur 1 gezeigte Ausführungsbeispiel, wobei sich die Sterilglocke in ihrer Sterilstellung befindet,
- Figur 3: einen Vertikalschnitt durch das in Figur 1 gezeigte Ausführungsbeispiel während des Befüllens,
- Figur 4: eine Draufsicht auf den Greifer des in Figur 1 gezeigten Ausführungsbeispiels,
- Figur 5: einen Vertikalschnitt durch ein Ausführungsbeispiel einer erfindungsgemäßen Sterilisiervorrichtung, wobei sich die Sterilglocke in ihrer Bereitschaftsstellung befindet,
- Figur 6: einen Vertikalschnitt durch das in Figur 5 gezeigte, erfindungsgemäße Ausführungsbeispiel mit der Sterilglocke in ihrer Sterilstellung,
- Figur 7: einen Vertikalschnitt durch das in Figur 5 gezeigte Ausführungsbeispiel der Sterilisiervorrichtung während des Befüllens,
- Figur 8: eine Draufsicht auf das Hebelelement des in den Figuren 5 bis 7 gezeigten Ausführungsbeispieles, und
- Figur 9: einen Vertikalschnitt durch ein weiteres Ausführungsbeispiel einer nicht erfindungsgemäßen Sterilisiervorrichtung.

Der Übersichtlichkeit halber sind in den Figuren nur Ausschnitte aus den Sterilisiervorrichtungen dargestellt, insbesondere nur die Bereiche zum Sterilisieren jeweils eines Behälters. Die Sterilisiervorrichtung selbst kann eine Vielzahl solcher Bereiche umfassen. Gleiche Komponenten sind in den Zeichnungen jeweils mit gleichen Bezugszeichen versehen.

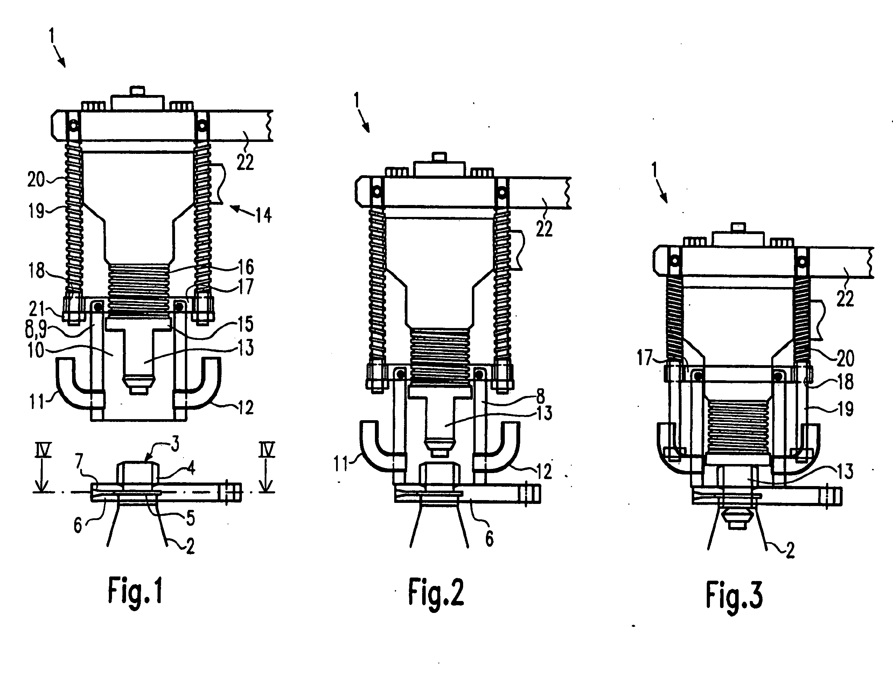

Figur 1 zeigt einen Ausschnitt eines Ausführungsbeispieles einer nicht erfindungsgemäßen Sterilisiervorrichtung 1. Die zu sterilisierenden und zu befüllenden Behälter 2 sind hier Flaschen. Es kann sich beispielsweise um Glasflaschen oder PET-Flaschen handeln. Sterilisiert werden können jedoch auch Flaschen aus anderen Materialien, beispielsweise aus anderen Kunststoffen oder Aluminium.

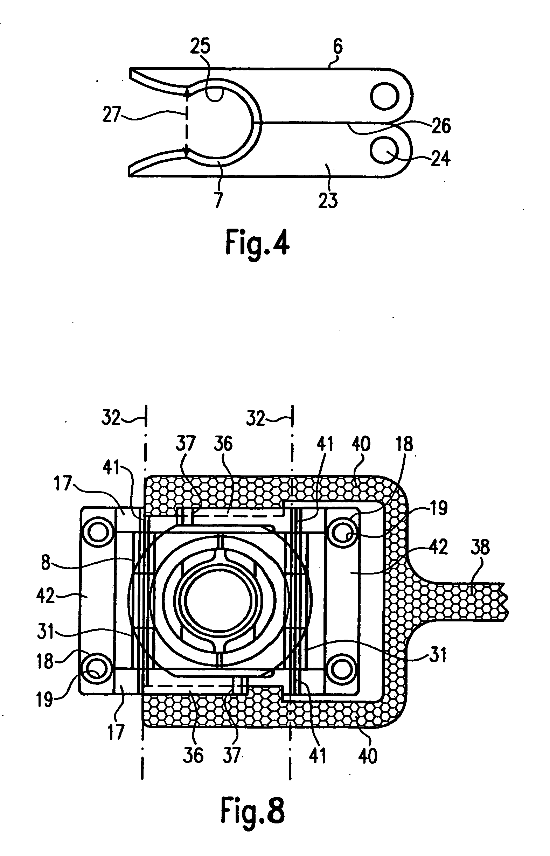

Der Behälter 2 weist eine Befüllöffnung 3 und einen Hals 4 mit einem nach außen vorstehenden Kragen 5 auf. An diesem Hals 4 ist der Behälter 2 von einem Positionierelement 6 erfasst, das hier als Greifer ausgebildet ist. Es verfügt über eine Nut 7 zur Aufnahme des Kragens 5 des Behälters. Zu einem äußeren Ende hin weitet sich die Nut 7, um die Aufnahme eines Kragens 5 zu erleichtern. Weitere Einzelheiten sind in der in Figur 4 gezeigten Draufsicht zu erkennen.

Mittels des Positionierelementes 6 wird der Behälter 2 so gehalten, dass er koaxial zu einer Sterilglocke 8 ausgerichtet ist. Die Sterilglocke 8 ist im vorliegenden Ausführungsbeispiel einstückig ausgebildet. Als Wand weist sie einen Zylindermantel 9 auf, der in seinem Inneren eine Sterilkammer 10 umfasst. An der Außenseite des Zylindermantels 9 sind ein Zuführstutzen 11 und ein Abführstutzen 12 angeformt. Mittels des Zuführstutzens, der beispielsweise durch einen übergestülpten, flexiblen Schlauch verlängert werden kann, wird Sterilisiergas in die Sterilglocke 8 eingeleitet. Durch den Abführstutzen 12 ist die Sterilkammer 10 wieder entleerbar.

Im Inneren der Sterilglocke 8 ist ein Füllkopf 13 einer Füllvorrichtung 14 aufgenommen. Der Füllkopf 13 verfügt über einen Flansch 15, der einen kreisförmigen Durchmesser hat und gleitend an der Innenseite des Zylindermantels 9 der Sterilglocke 8 anliegt. Nach oben hin ist die Sterilkammer 10 daher durch den Flansch 15 abgedichtet. Der Füllkopf 13 ist koaxial zum Zylindermantel 9 und damit auch zur Befüllöffnung 3 gelagert. Mit seinem Flansch 15 ist er an einem Gewinde 16 angeschraubt, so dass seine Höheneinstellung variiert werden kann.

Die Sterilglocke 8 ist an zwei Querträger 17 befestigt, von denen der eine gezeigt ist. Die Querträger 17 verfügen an ihren Enden über Führungsöffnungen 18. Durch jede Führungsöffnung 18 erstreckt sich eine an der Füllvorrichtung 14 befestigte Führung 19. Hier sind als Führungen 19 vier Zylinderstangen vorgesehen. Über jede Zylinderstange liegt eine spiralförmige Druckfeder 20, die den Querträger 17 beaufschlagt und ihn gegen einen Anschlag 21 am Ende der Führung 19 zwingt. Die Zylinderstangen der Führung 19 sind geradlinig und sie sind insbesondere parallel zur Achse des Zylindermantels 9 der Sterilglocke 8 ausgebildet.

Die Füllvorrichtung 14 ist an einer Aufhängung 22 befestigt. Auch die Führung 19 ist an derselben Aufhängung befestigt. Die Höhe der Aufhängung 22 ist veränderbar. In Figur 1 ist sie so angeordnet, dass die Sterilglocke 8 auf ihrer Unterseite offen ist. Damit befindet sich die Sterilglocke 8 in ihrer Bereitschaftsstellung, in der ein Abschnitt eines zu sterilisierenden Behälters 2, insbesondere dessen Befüllöffnung 3 in der Sterilglocke 8 aufnehmbar ist. Hier kann der Behälter 2 von unten in die Sterilglocke 8 hineingeführt werden.

Gegenüber der in Figur 1 gezeigten Stellung ist die Aufhängung 22 in Figur 2 um einen definierten Weg nach unten gefahren worden. Die Unterseite der Sterilglocke 8 beaufschlagt die Arme des Positionierelementes 6. Die Sterilglocke 8 befindet sich in ihrer Sterilstellung. Wird Sterilisiergas über den Zuführstutzen 11 in die Sterilkammer 10 eingeführt, so ist das Gas durch den Kontakt zwischen Sterilglocke 8 und Positionierelement 6 am Ausfließen aus der Sterilkammer 10 gehindert. Statt dessen wird das Sterilisiergas in das Innere des Behälters 2 hineinfließen. Über den Abführstutzen 12 kann überschüssiges oder verdampftes Sterilisiergas aus der Sterilkammer 10 abgeführt oder gegebenenfalls abgesaugt werden.

Der Füllkopf 13 befindet sich in Figur 2 in seiner Wartestellung. Zwischen seiner Unterseite und der Befüllöffnung 3 des Behälters 2 verbleibt ein Spalt, durch den das Sterilisiergas in den Behälter 2 gelangt.

In Figur 3 ist die Sterilisiervorrichtung in einer Stellung gezeigt, in der die Aufhängung 22 gegenüber der in Figur 2 gezeigten Stellung noch weiter nach unten gefahren ist. Der Füllkopf 13 hat sich dabei durch die Befüllöffnung 3 in den Behälter 2 hineinbewegt und befindet sich nun in seiner Füllstellung. Durch einen Füllkanal (nicht dargestellt) im Inneren des Füllkopfes 13 kann der Behälter 2 nun befüllt werden.

Der Zylindermantel 9 der Sterilglocke 8 liegt auf dem Positionierelement 6 auf. Da das Positionierelement 6 einen Widerstand gegen eine weitere Bewegung der Sterilglocke 8 darstellt, bewegt sich bei der Abwärtsbewegung der Aufhängung 22 der Querträger 17 über die Führung 19 und staucht dabei die Druckfeder 20. Die Druckfeder 20 übt nun eine Kraft auf den Querträger 17 aus, die auf die Sterilglocke 8 übertragen wird und diese an das Positionierelement 6 andrückt.

In Figur 4 ist eine Draufsicht auf den Greifer 6 der Sterilisiervorrichtung 1 in der in Figur 1 mit IV-IV bezeichneten Ebene. Der Greifer, der hier das Positionierelement 6 bildet, weist zwei Greifarme 23 auf. Jeder der Greifarme 23 ist um eine Schwenkachse 24 schwenkbar. An einem der Schwenkachse 24 gegenüberliegenden Ende jedes Greifarms 23 ist eine Aussparung 25 vorgesehen, die der Form des Halses 4 eines Behälters 2 angepasst ist. Entlang der Aussparung 25 erstreckt sich die Nut 7, in der der Kragen 5 eines Behälters 2 aufnehmbar ist.

In der dargestellten Schließstellung liegen die Greifarme 23 an einer gemeinsamen Kontaktebene 26 aneinander an. Eine Verengung 27, die kürzer ist als der Durchmesser des Behälterhalses 4, hindert den Behälter 2 am seitlichen Ausbrechen aus der Aussparung 25.

Bei diesem Ausführungsbeispiel einer Sterilisiervorrichtung 1 stellt die verfahrbare Aufhängung 22 das Schließelement dar. Die Aufhängung 22 sorgt dafür, dass die Sterilglocke 8 auf den Greifer 6 aufgesetzt wird und dabei die Befüllöffnung 3 umgibt, d.h. das Verfahren der Aufhängung 22 sorgt für das Überführen der Sterilglocke 3 aus deren Bereitschaftsstellung in die Sterilstellung.

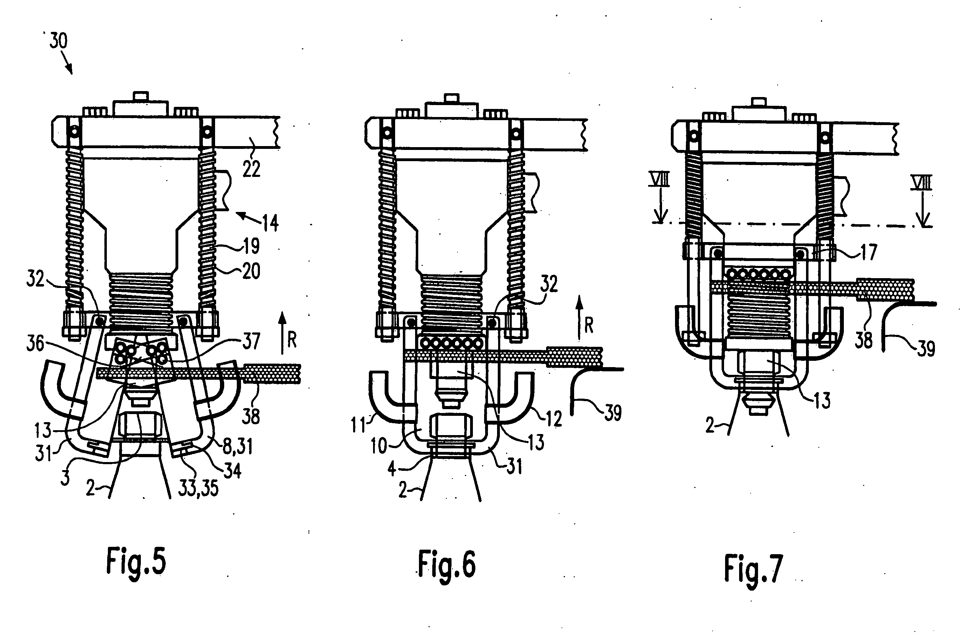

In Figur 5 ist ein Ausschnitt aus einem Ausführungsbeispiel einer erfindungsgemäßen Sterilisiervorrichtung 30 dargestellt. Gegenüber dem ersten Ausführungsbeispiel sind die Füllvorrichtung 14 mit der Aufhängung 22 und die Führung 19 unverändert. Anders als im ersten Ausführungsbeispiel ist die Sterilglocke 8 im erfindungsgemäßen Ausführungsbeispiel jedoch zweiteilig, da sie zwei Wandelemente 31 aufweist. Jedes Wandelement 31 ist im Wesentlichen schaufelförmig ausgebildet und um eine horizontale Schwenkachse 32 schwenkbar. Am unteren Ende jedes Wandelementes 31 ist eine Aussparung 33 vorgesehen, an der der Hals 4 eines Behälters 2 aufnehmbar ist. Auf der Innenseite der Aussparung 33 ist zudem eine Nut 34 angeordnet, die zur Aufnahme des Kragens 5 eines Behälters 2 dient. Die Aussparung 33 und die Nut 34 stellen hier das Positionierelement 35 für einen Behälter 2 dar.

Auf der Außenseite jedes Wandelementes 31 ist ein Hebelkörper 36 befestigt. An seinem Ende befindet sich ein Reibungsminderer 37, der hier als Rolle ausgebildet ist. Ein gabelförmiges Hebelelement 38 umfasst die Sterilglocke 8 wenigstens teilweise. Es ist in einer vertikalen Richtung R gegenüber der Füllvorrichtung 14 bewegbar.

In Figur 5 befindet sich die Sterilkammer 8 der Sterilisiervorrichtung 30 in ihrer Bereitschafts-Stellung. Die Wandelemente 31 sind nach außen verschwenkt und können so zwischen sich einen Behälter 2, insbesondere dessen Befüllöffnung 3, aufnehmen. Die Wandelemente 31 können so gegeneinander vorgespannt sein, dass sie von sich aus diese Bereitschafts-Stellung einnehmen.

In Figur 6 befindet sich die Sterilglocke 8 in ihrer Sterilstellung. Um sie in diese Stellung zu bringen, ist das Hebelelemente 38 auf eine Kurvensteuerung 39 aufgeglitten und wurde dadurch in Richtung R nach oben bewegt. Die Hebelkörper 36 sind an der Oberseite des Hebelelementes 38 entlanggeglitten, wobei die Reibung durch den Reibungsminderer 37 verringert wurde. Die Hebelkörper 36 befinden sich in Figur 6 in einer horizontalen Lage. Durch ihr Verschwenken haben sie die Wandelemente 31 um die Schwenkachsen 32 verschwenkt. Dabei haben sich die schaufelartigen Wandelemente 31 so um einen Behälter 2 geschlossen, dass dieser an seinem Hals ergriffen wurde und nun durch das Positionierelement 35 festgelegt ist.

Die Sterilkammer 8 befindet sich in ihrer Sterilstellung, in der sie die Befüllöffnung 3 des Behälters und einen sich daran anschließenden Abschnitt des Behälters 2 umgibt. Bis auf den Zuführstutzen 11 und den Abführstutzen 12 bestehen keine Öffnungen der Sterilkammer 10 innerhalb der Sterilglocke 8 nach außen. Nach oben hin ist die Sterilkammer 10 durch den Flansch 15 des Füllkopfes 13 abgedichtet. Zwischen dem Füllkopf und der Füllöffnung 3 des Behälters 2 besteht ein Spalt, durch den Sterilisiergas in den Behälter 2 hineingelangen kann.

In diesem erfindungsgemäßen Ausführungsbeispiel einer Sterilisiervorrichtung 30 bildet das Hebelelement 38 das Schließelement, das die Sterilglocke 8 von ihrer Bereitschaftsstellung in die Sterilstellung überführt.

Figur 7 zeigt die Sterilisiervorrichtung 30 in einer Stellung, in der das Hebelelement 38 noch weiter auf der Kurvensteuerung 39 nach oben geführt wurde. Während dieser Bewegung liegen die Hebelkörper 36 der beiden Wandelemente 31 auf der Oberseite des Hebelelementes 38 auf. Das Verfahren des Hebelelementes 38 führt daher zum Anheben der gesamten Sterilglocke 8. Da das Positionierelement 39 an der Sterilglocke 8 angeformt ist, wird auch der Behälter 2 mit nach oben geführt. Der Querträger 17, an dem sich die Schwenklager der Schwenkachsen 32 der Wandelemente 31 befinden, bewegt sich entlang der Führungen 19 nach oben und deformiert dabei die Druckfeder 20. Da die Sterilglocke 8 relativ zu der Füllvorrichtung 14 verfährt, bewegt sich der Füllkopf 13 relativ zum Behälter 2. Der Behälter 2 wird mit seiner Befüllöffnung 3 auf den Füllkopf 13 hinaufgefahren, so dass sich der Füllkopf 13 zumindest abschnittsweise in den Behälter 2 hinein erstreckt. In dieser Füllstellung kann der Behälter 2 gefüllt werden.

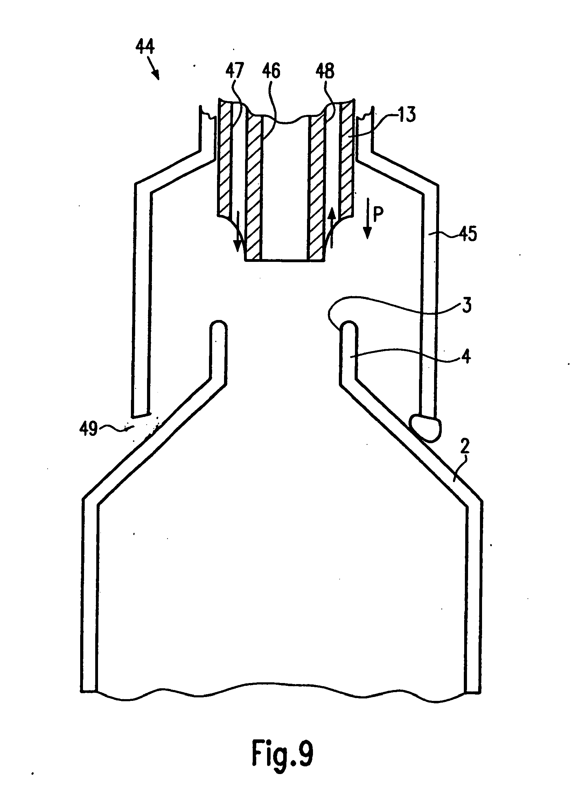

Figur 8 zeigt einen Vertikalschnitt durch das erfindungsgemäße Ausführungsbeispiel einer Sterilisiervorrichtung 30 in der in Figur 7 mit VIII-VIII bezeichneten Ebene. Der Übersichtlichkeit halber sind einige Komponenten nicht dargestellt, wie beispielsweise der Zuführstutzen 11 oder der Abführstutzen 12. Zu sehen ist insbesondere das Hebelelement 38, das hier gabelförmig mit zwei Gabelarmen 40 ausgebildet ist. Zwischen den Gabelarmen 40 befindet sich die Sterilglocke 8. Ihre beiden Wandelemente 31 sind um Schwenkachsen 32 verschwenkbar. Die Schwenklager 41 zu diesen Schwenkachsen 32 sind in Querträger 17 oberhalb der Sterilglocke 8 untergebracht.

Zwei Querträger 17 sind jeweils durch Querstreben 42 miteinander verbunden, so dass sie ein rechteckiges Tragegerüst bilden. In den Querstreben 42 sind Führungsöffnungen 18 vorgesehen, in denen jeweils eine Zylinderstange als Führung 19 aufgenommen ist. Die vier Führungen 19 sind geradlinig und parallel zueinander ausgerichtet. Jedes der beiden Wandelemente 31 der Sterilglocke 8 weist auf seiner Außenseite einen Hebelkörper 36 auf, der sich senkrecht zur Schwenkachse 32 in Verlängerung einer Wand des Wandelementes 31 erstreckt. An einem Ende jedes Hebelkörpers 36 ist ein Reibungsminderer 37 in Form einer Rolle vorgesehen, die auf der Oberseite des ihr zugeordneten Gabelarmes 40 abrollen kann. Der untere Hebelkörper 36 ist dem linken Wandelement 31 zugeordnet, während der obere Hebelkörper 36 am rechten Wandelemente 31 angeformt ist.

Figur 9 zeigt einen Vertikalschnitt durch ein weiteres Ausführungsbeispiel einer nicht erfindungsgemäßen Sterilisiervorrichtung 44. Gegenüber den ersten beiden Ausführungsbeispielen ist insbesondere die Sterilglocke 45 stark vereinfacht. Sie ist einteilig ausgebildet und weist keine Zuführstutzen 11 oder Abführstutzen 12 mehr auf. Stattdessen sind im Füllkopf 13 zusätzlich zum Füllkanal 46 ein Zuführkanal 47 zum Zuführen des Sterilisiergases und ein Abführkanal 48 vorgesehen. Der Füllkopf 13 ist gegenüber der Sterilglocke 45 an deren Längsrichtung bewegbar. Er kann auf den Behälter 2 an dessen Befüllöffnung 3 aufgesetzt werden.

Der Behälter 2 wird von einem nicht dargestellten Positionierelement gehalten und positioniert. Dabei kann das Positionierelement beispielsweise ein Teller oder ein Greifer sein. Mittels des Positionierelementes wird der Behälter 2 gegen die Sterilglocke 45 angehoben, bis der Hals des Behälters 2 in die Sterilglocke 45 hineinragt. Das bewegbare Positionierelement stellt daher das Schließelement der Sterilisiervorrichtung 44 dar.

In der in Figur 9 gezeigten Sterilstellung liegt der untere Rand der Sterilglocke 45 teilweise auf dem Behälter 2 auf. Allerdings ist die Sterilkammer nicht gegenüber dem Behälter 2 abgedichtet, so dass am unteren Rand der Sterilglocke 45 Sterilisiergas durch einen Spalt 49 ausströmen kann. Das in die Sterilglocke 45 eingeleitete Sterilisiergas sorgt für einen leichten Überdruck innerhalb der Sterilkammer, der das Eindringen von nicht-steriler Umgebungsluft verhindert. Der Füllkopf 13 wird von der dargestellten Sterilstellung in die Füllstellung gebracht, in dem er entlang der durch den Pfeil P bezeichneten Richtung in die Befüllöffnung 3 hineingefahren wird.

Die erfindungsgemäße Sterilisiervorrichtung kann eine Transportvorrichtung aufweisen, beispielsweise einen Rundläufer. An diesem befinden sich eine Vielzahl von Positionierelementen 6 für Behälter 2, wobei die Positionierelemente 6 vorzugsweise als Greifer ausgebildet sind. In einem bevorzugten Ausführungsbeispiel ist jedem Positionierelement eine Sterilglocke und eine Füllvorrichtung zugeordnet.

Im Folgenden wird der Betriebsablauf einer erfindungsgemäßen Sterilisiervorrichtung 30 kurz geschildert.

Von einer Zuliefervorrichtung werden zu sterilisierende und zu befüllende Behälter 2 angeliefert. Der vorderste Behälter 2 auf der Zuliefervorrichtung wird von einem als Greifer ausgebildeten Positionierelement 6 ergriffen und positioniert. Dabei kann sich der Greifer 6 an einer Transportvorrichtung befinden, so dass der ergriffene Behälter 2 eine Bewegung entlang der Transportvorrichtung ausführt.

Setzt sich das Positionierelement 35 aus Wandelementen 31 der Sterilglocke 8 zusammen, so ist das Ergreifen des Behälters 2 gleichbedeutend mit dem Schließen der Sterilglocke 8, d.h. dem Überführen der Sterilglocke 8 von ihrer Bereitschafts-Stellung in ihre Sterilstellung (vergleiche Figuren 5 bis 7).

Andemfalls werden der ergriffene Behälter 2 und die Sterilglocke 8, 45 zueinander geführt. In der Sterilstellung umgibt die Sterilglocke 8, 45 die Befüllöffnung 3 des Behälters 2 sowie den Füllkopf 13. Zwischen dem Füllkopf 13 und der Befüllöffnung 3 besteht Spalt ein. Sterilisiergas wird durch eine Zuführöffnung, beispielsweise einen Zuführstutzen 11 oder einen Zuführkanal 47, in die Sterilkammer 10 innerhalb der Sterilglocke 8 eingeleitet, gelangt in den Behälter 2 und kondensiert an dessen Innenwand, da der Behälter 2 auf einer niedrigeren Temperatur gehalten wird, als das Sterilisiergas. Es bildet sich ein bakterientötendes und daher sterilisierendes Kondensat.

Im nächsten Schritt wird durch die Zuführöffnung Heißluft in die Sterilkammer 10 eingeblasen. Das Kondensat verdampft und der Innenraum des Behälters 2 trocknet. Das verdampfte Sterilisier-Kondensat und das nicht kondensierte Sterilisiergas werden durch eine Abführöffnung, beispielsweise einen Abführstutzen 12 oder einen Abführkanal 48, aus der Sterilkammer 10 ausgeblasen.

Sobald das Kondensat im Behälter 2 vollständig getrocknet, beziehungsweise ausgeblasen ist, wird die Sterilisiervorrichtung 30 in ihre Füllstellung gebracht. Dazu wird der Füllkopf 13 zur Befüllöffnung 3 des Behälters 2 bewegt. Alternativ bewegt sich der Behälter 2 mit der Sterilglocke 8, 45 relativ zum Füllkopf 13, bis der Füllkopf 13 den Behälter 2 beaufschlagt. Über den Füllkopf 13, insbesondere den Füllkanal 46, wird der Behälter 2 befüllt. Damit sind das Sterilisieren und Befüllen des Behälters abgeschlossen und der Behälter 2 muss nur noch verschlossen werden.

Bei der Inbetriebnahme der Sterilisiervorrichtung findet zunächst eine Vorsterilisierung der Sterilglocke statt. Dabei wird die Sterilglocke um die Öffnung eines Dummy-Behälters geschlossen. Nachdem die Sterilglocke mittels Heißluft vorgetrocknet wurde, wird Sterilisiergas in die Sterilglocke eingeleitet. Das Gas kondensiert an der Innenwand der Sterilglocke und trocknet bei der Einleitung weiterer Heißluft. Sobald die Sterilglocke sterilisiert ist, kann der Dummy-Behälter entfemt werden und der Sterilisier- und Abfüllprozess für die Behälter anlaufen.

Ausgehend von den dargestellten und beschriebenen Ausführungsbeispielen kann die erfindungsgemäße Sterilisiervorrichtung in vielfacher Hinsicht abgewandelt werden. Statt an der Füllvorrichtung 14 - wie in den dargestellten Ausführungsbeispielen - kann beispielsweise die Sterilglocke auch am Greifer 6 vorgesehen werden. Dabei würden zwei Greifarme des Greifers die Befüllöffnung 3 des Behälters 2 als Sterilglocke umgeben und den Behälter 2 dann gegen die Füllvorrichtung 14, beziehungsweise deren Füllkopf 13 bewegen. Die Sterilglocke könnte auch mehr als zwei Wandelemente umfassen.

## Patentansprüche

1. Sterilisiervorrichtung (30) für eine Befüllöffnung (3) aufweisende Behälter (2), insbesondere für Flaschen, die mindestens eine Sterilkammer (10) zur Aufnahme eines Sterilisiergases sowie mindestens eine Füllvorrichtung (14) zum Befüllen der sterilisierten Behälter mittels eines Füllkopfes (13) aufweist, ferner mit einer Sterilglocke (8, 45), die mittels eines Schließelementes (22, 38) von einer Bereitschafts-Stellung zu einer Sterilstellung überführbar ist, in der die Sterilglocke (8, 45) den Füllkopf (13) und die Befüllöffnung (3) des Behälters (2) umgibt und in der die Sterilkammer (10) von der Sterilglocke (8, 45), dem Füllkopf (13) und der Befüllöffnung (3) des Behälters (2) begrenzt ist, **dadurch gekennzeichnet, dass** die Sterilglocke (8) zwei oder mehrere Wandelemente (31) aufweist, die relativ zueinander verschwenkbar sind.

2. Sterilisiervorrichtung nach Anspruch 1, **dadurch gekennzeichnet, dass** die Sterilglocke (8, 45) durch Einleiten des Sterilisiergases sterilisierbar ist.

3. Sterilisiervorrichtung nach Anspruch 1 oder 2, **dadurch gekennzeichnet, dass** die Sterilglocke (8, 45) während einer Befüllung des Behälters (2) sterilisierbar ist, indem permanent das Sterilisiergas zuführbar ist.

4. Sterilisiervorrichtung nach wenigstens einem der vorangehenden Ansprüche, **dadurch gekennzeichnet, dass** die Sterilglocke (8, 45) in ihrer Sterilstellung die Befüllöffnung (3) des Behälters (2) teilweise, nämlich unter Belassung mindestens eines Spülspaltes (49), schließend umgibt.

5. Sterilisiervorrichtung nach wenigstens einem der vorangehenden Ansprüche, **dadurch gekennzeichnet, dass** in der Sterilstellung zum Austreten eines geringen Anteiles des Sterilisiergases aus der Sterilglocke (8, 45) nach außen ein Spülspalt (49) zwischen der Sterilglocke und dem Behälter (2) vorgesehen ist.

6. Sterilisiervorrichtung nach wenigstens einem der vorangehenden Ansprüche, **dadurch gekennzeichnet, dass** mittels des Sterilisiergases innerhalb der Sterilglocke (8, 45) gegenüber ihrer Umgebung ein leichter Überdruck herstellbar ist.

7. Sterilisiervorrichtung nach wenigstens einem der vorangehenden Ansprüche, **dadurch gekennzeichnet, dass** die beiden Wandelemente (31) auf die Bereitschafts-Stellung der Sterilglocke (8, 45) hin gegeneinander vorgespannt sind.

8. Sterilisiervorrichtung nach wenigstens einem der vorangehenden Ansprüche, **dadurch gekennzeichnet, dass** mit jedem bewegbaren Wandelement (31) ein Hebelkörper (36) verbunden ist, der durch ein Hebelelement (38) beaufschlagbar ist.

9. Sterilisiervorrichtung nach wenigstens Anspruch 8, **dadurch gekennzeichnet, dass** das Hebelelement (38) gabelförmig ist.

10. Sterilisiervorrichtung nach wenigstens einem der Ansprüche 8 oder 9, **dadurch gekennzeichnet, dass** an dem Hebelkörper (36) ein Reibungsminderer (37) zum Abgleiten oder Abrollen am Hebelelement (38) vorgesehen ist.

11. Sterilisiervorrichtung nach wenigstens einem der vorangehenden Ansprüche, **dadurch gekennzeichnet, dass** die Sterilglocke (8, 45) an der Füllvorrichtung (14) angeordnet ist.

12. Sterilisiervorrichtung nach wenigstens einem der vorangehenden Ansprüche, **dadurch gekennzeichnet, dass** die Sterilglocke (8, 45) zumindest in ihrer Sterilstellung durch die Füllvorrichtung (14), insbesondere durch den Füllkopf (13), abgedichtet ist.

13. Sterilisiervorrichtung nach wenigstens einem der vorangehenden Ansprüche, **dadurch gekennzeichnet, dass** der Füllkopf (13) relativ zur Sterilglocke (8, 45) in deren Sterilstellung bewegbar ist.

14. Sterilisiervorrichtung nach wenigstens einem der vorangehenden Ansprüche, **dadurch gekennzeichnet, dass** die Sterilglocke (8, 45) in ihrer Sterilstellung relativ zur Füllvorrichtung (14) entlang einer Führung (19) bewegbar ist.

15. Sterilisiervorrichtung nach Anspruch 14 in Verbindung mit zumindest Anspruch 8, **dadurch gekennzeichnet, dass** die Führung (19) geradlinig ist und die mittels des Hebelelementes (38) auf einen Hebelkörper (36) ausgeübte Kraft etwa parallel zu der Führung (19) gerichtet ist.

16. Sterilisiervorrichtung nach wenigstens einem der vorangehenden Ansprüche, **dadurch gekennzeichnet, dass** die Sterilglocke (8, 45) gegenüber der Füllvorrichtung (14) federnd gelagert ist.

17. Sterilisiervorrichtung nach wenigstens einem der vorangehenden Ansprüche, **gekennzeichnet durch** mindestens ein Positionierelement (6, 35) zum Positionieren eines Behälters (2) gegenüber der Füllvorrichtung (14).

18. Sterilisiervorrichtung nach Anspruch 17, **dadurch gekennzeichnet, dass** das mindestens eine Positionierelement (6, 35) ein Greifer ist.

19. Sterilisiervorrichtung nach wenigstens einem der Ansprüche 17 oder 18, **dadurch gekennzeichnet, dass** das Positionierelement (6) durch die Sterilglocke (8, 45) in deren Sterilstellung beaufschlagbar ist.

20. Sterilisiervorrichtung nach wenigstens einem der Ansprüche 17 bis 19, **dadurch gekennzeichnet, dass** das Positionierelement (35) an der Sterilglocke (8, 45) angeordnet ist.

21. Sterilisiervorrichtung nach Anspruch 20, **dadurch gekennzeichnet, dass** das Positionierelement (35) an der Sterilglocke (8, 45) als Aussparung (33) zur Aufnahme eine Behälterabschnittes ausgebildet ist.

22. Sterilisiervorrichtung nach wenigstens einem der Ansprüche 17 bis 21, **dadurch gekennzeichnet, dass** das Positionierelement (35, 6) relativ zur Füllvorrichtung (14) verfahrbar ist.

23. Sterilisiervorrichtung nach wenigstens einem der Ansprüche 17 bis 22, **dadurch gekennzeichnet, dass** der Füllkopf (13) relativ zum Positionierelement (6, 35) verfahrbar ist.

24. Sterilisiervorrichtung nach wenigstens einem der Ansprüche 17 bis 23, **dadurch gekennzeichnet, dass** jedem Positionierelement (6) genau eine Sterilglocke (8, 45) zugeordnet ist.

25. Sterilisiervorrichtung nach wenigstens einem der Ansprüche 17 bis 24, **dadurch gekennzeichnet, dass** jedem Positionierelement (6, 35) und/oder jeder Sterilglocke (8, 45) genau ein Füllkopf (13) zugeordnet ist.

26. Sterilisiervorrichtung nach wenigstens einem der Ansprüche 17 bis 25, **dadurch gekennzeichnet, dass** das Positionierelement (6) und das Schließelement identisch sind, indem der zu sterilisierende Behälter (2) mittels des Positionierelement (6) in eine Sterilstellung verfahrbar ist, in der die Sterilglocke (8, 45) die Befüllöffnung (3) des Behälters (2) umgibt.

27. Sterilisiervorrichtung nach wenigstens einem der vorangehenden Ansprüche, **gekennzeichnet durch** eine Kurvensteuerung (39) zum Steuern des Schließelementes (6, 38).

28. Sterilisiervorrichtung nach wenigstens Anspruch 27, **dadurch gekennzeichnet, dass** die Kurvensteuerung (39) zum Steuern des Schließelementes an einer Transportvorrichtung für die zu sterilisierenden Behälter (2) vorgesehen ist.

## Claims

1. Sterilization device (30) for containers (2) that have a filling opening (3), in particular for bottles, said sterilization device (30) comprising at least one sterile chamber (10) for receiving a sterilization gas, and at least one filler device (14) for filling the sterilized containers via a filler head (13), and in addition with a sterile bell (8, 45) which can be converted by means of a closure element (22, 38) from a standby position to a sterile position in which the sterile bell (8, 45) surrounds the filler head (13) and the filling opening (3) of the container (2) and in which the sterile chamber (10) is delimited by the sterile bell (8, 45), the filler head (13) and the filling opening (3) of the container (2), **characterized in that** the sterile bell (8) has two or more wall elements (31) which are able to pivot relative to one another.

2. Sterilization device according to Claim 1, **characterized in that** the sterile bell (8, 45) can be sterilized by introduction of the sterilization gas.

3. Sterilization device according to Claim 1 or 2, **characterized in that** the sterile bell (8, 45) can be sterilized by means of a continuous delivery of the sterilization gas while the container (2) is being filled.

4. Sterilization device according to at least one of the preceding claims, **characterized in that** the sterile bell (8, 45), in its sterile position, surrounds the filling opening (3) of the container (2) partially, that is to say leaving at least one flushing gap (49).

5. Sterilization device according to at least one of the preceding claims, **characterized in that**, in the sterile position, in order to allow a small proportion of the sterilization gas to escape outwards from the sterile bell (8, 45), a flushing gap (49) is provided between the sterile bell and the container (2).

6. Sterilization device according to at least one of the preceding claims, **characterized in that**, by means of the sterilization gas, a slight overpressure can be created inside the sterile bell (8, 45) in relation to its environment.

7. Sterilization device according to at least one of the preceding claims, **characterized in that** the two wall elements (31) are mutually pretensioned towards the standby position of the sterile bell (8, 45).

8. Sterilization device according to at least one of the preceding claims, **characterized in that** a lever body (36), that can be acted upon by a lever element (38), is connected to each movable wall element (31).

9. Sterilization device according to at least Claim 8, **characterized in that** the lever element (38) is fork-shaped.

10. Sterilization device according to at least one of Claims 8 and 9, **characterized in that** a friction reducer (37) is provided on the lever body (36) for sliding or rolling on the lever element (38).

11. Sterilization device according to at least one of the preceding claims, **characterized in that** the sterile bell (8, 45) is arranged on the filler device (14).

12. Sterilization device according to at least one of the preceding claims, **characterized in that** the sterile bell (8, 45), at least in its sterile position, is sealed off by the filler device (14), in particular by the filler head (13).

13. Sterilization device according to at least one of the preceding claims, **characterized in that** the filler head (13) is movable relative to the sterile bell (8, 45) in the sterile position thereof.

14. Sterilization device according to at least one of the preceding claims, **characterized in that** the sterile bell (8, 45), in its sterile position, can be moved relative to the filler device (14) along a guide (19).

15. Sterilization device according to Claim 14 in combination with at least Claim 8, **characterized in that** the guide (19) is rectilinear and the force exerted by the lever element (38) on a lever body (36) is oriented approximately parallel to the guide (19).

16. Sterilization device according to at least one of the preceding claims, **characterized in that** the sterile bell (8, 45) is mounted with a spring action relative to the filler device (14).

17. Sterilization device according to at least one of the preceding claims, **characterized by** at least one positioning element (6, 35) for positioning a container (2) relative to the filler device (14).

18. Sterilization device according to Claim 17, **characterized in that** the at least one positioning element (6, 35) is a gripper.

19. Sterilization device according to at least one of Claims 17 and 18, **characterized in that** the positioning element (6) can be acted upon by the sterile bell (8, 45) in the sterile position thereof.

20. Sterilization device according to at least one of Claims 17 to 19, **characterized in that** the positioning element (35) is arranged on the sterile bell (8, 45).

21. Sterilization device according to Claim 20, **characterized in that** the positioning element (35) on the sterile bell (8, 45) is designed as a recess (33) for receiving a container section.

22. Sterilization device according to at least one of Claims 17 to 21, **characterized in that** the positioning element (35, 6) can be driven relative to the filler device (14).

23. Sterilization device according to at least one of Claims 17 to 22, **characterized in that** the filler head (13) can be driven relative to the positioning element (6, 35).

24. Sterilization device according to at least one of Claims 17 to 23, **characterized in that** each positioning element (6) is assigned exactly one sterile bell (8, 45).

25. Sterilization device according to at least one of Claims 17 to 24, **characterized in that** each positioning element (6, 35) and/or each sterile bell (8, 45) is assigned exactly one filler head (13).

26. Sterilization device according to at least one of Claims 17 to 25, **characterized in that** the positioning element (6) and the closure element are identical, since the container (2) to be sterilized can be driven by means of the positioning element (6) into a sterile position in which the sterile bell (8, 45) surrounds the filling opening (3) of the container (2).

27. Sterilization device according to at least one of the preceding claims, **characterized by** a cam control (39) for controlling the closure element (6, 38).

28. Sterilization device according to at least Claim 27, **characterized in that** the cam control (39) for controlling the closure element is provided on a transport device for the containers (2) that are to be sterilized.

## Revendications

1. Dispositif de stérilisation (30) pour un récipient (2) présentant une ouverture de remplissage (3), en particulier pour des bouteilles, présentant au moins une chambre stérile (10) pour recevoir un gaz de stérilisation, ainsi qu'au moins un dispositif de remplissage (14) pour remplir les récipients stérilisés au moyen d'une tête de remplissage (13), en outre avec une cloche stérile (8, 45), susceptible d'être passée, au moyen d'un élément de fermeture (22, 38), d'une position de disponibilité à une position stérile, à laquelle la cloche stérile (8, 45) entoure la tête de remplissage (13) et l'ouverture de remplissage (3) du récipient (2) et dans laquelle la chambre de stérilisation (10) est délimitée par la cloche stérile (8, 45), la tête de remplissage (13) et l'ouverture de remplissage (3), **caractérisé en ce que** la cloche stérile (8) présente deux éléments de paroi (31) ou plus, susceptibles de pivoter les uns par rapport aux autres.

2. Dispositif de stérilisation selon la revendication 1, **caractérisé en ce que** la cloche stérile (8, 45) est stérilisable par introduction du gaz de stérilisation.

3. Dispositif de stérilisation selon la revendication 1 ou 2, **caractérisé en ce que** la cloche stérile (8, 45) est stérilisable pendant un remplissage du récipient (2), par le fait que le gaz de stérilisation peut être amené de façon permanente.

4. Dispositif de stérilisation selon au moins l'une des revendications précédentes, **caractérisé en ce que** la cloche stérile (8, 45) à position stérile entoure, avec effet de fermeture, partiellement l'ouverture de remplissage (3) du récipient (2), précisément en laissant subsister au moins un intervalle de remplissage (49).

5. Dispositif de stérilisation selon au moins l'une des revendications précédentes, **caractérisé en ce que**, à la position stérile, pour faire sortir une faible proportion du gaz de stérilisation de la cloche stérile (8, 45) vers l'extérieur, un intervalle de rinçage (49) est prévu, entre la cloche stérile et le récipient (2).

6. Dispositif de stérilisation selon au moins l'une des revendications précédentes, **caractérisé en ce que**, au moyen du gaz de stérilisation, à l'intérieur de la cloche stérile (8, 45), une légère surpression peut être établie par rapport à son environnement.

7. Dispositif de stérilisation selon au moins l'une des revendications précédentes, **caractérisé en ce que** les deux éléments de paroi (31) sont précontraints l'un contre l'autre, lorsque la cloche stérile (8, 45) est à la position de disponibilité.

8. Dispositif de stérilisation selon au moins l'une des revendications précédentes, **caractérisé en ce qu'**à chaque élément de paroi (31) déplaçable est relié un corps formant levier (36), pouvant être sollicité par un élément formant levier (38).

9. Dispositif de stérilisation selon au moins la revendication 8, **caractérisé en ce que** l'élément formant levier (38) est en forme de fourche.

10. Dispositif de stérilisation selon au moins l'une des revendications 8 ou 9, **caractérisé en ce que**, sur le corps de levier (36), est prévu un diminueur de frottement (37), pour faire glisser ou rouler sur l'élément de levier (38).

11. Dispositif de stérilisation selon au moins l'une des revendications précédentes, **caractérisé en ce que** la cloche stérile (8, 45) est disposée sur le dispositif de remplissage (14).

12. Dispositif de stérilisation selon au moins l'une des revendications précédentes, **caractérisé en ce que** la cloche stérile (8, 45) est isolée hermétiquement au moins à sa position stérile, au moyen du dispositif de remplissage (14), en particulier, au moyen du corps de remplissage (13).

13. Dispositif de stérilisation selon au moins l'une des revendications précédentes, **caractérisé en ce que** la tête de remplissage (13) est déplaçable par rapport à la cloche stérile (8, 45), à sa position stérile.

14. Dispositif de stérilisation selon au moins l'une des revendications précédentes, **caractérisé en ce que** la cloche stérile (8, 45) est déplaçable à sa position stérile par rapport au dispositif de remplissage (14), le long d'un guidage (19).

15. Dispositif de stérilisation selon la revendication 14, en liaison avec au moins la revendication 8, **caractérisé en ce que** le guidage (19) est rectiligne et la force, exercée sur un corps formant levier (36) au moyen de l'élément formant levier (38), est orientée à peu près parallèlement au guidage (19).

16. Dispositif de stérilisation selon au moins l'une des revendications précédentes, **caractérisé en ce que** la cloche stérile (8, 45) est montée élastiquement par rapport au dispositif de remplissage (14).

17. Dispositif de stérilisation selon au moins l'une des revendications précédentes, **caractérisé par** au moins un élément de positionnement (6, 35), pour le positionnement d'un récipient (2) par rapport au dispositif de remplissage (14).

18. Dispositif de stérilisation selon la revendication 17, **caractérisé en ce que** le au moins un élément de positionnement (6, 35) est une pince.

19. Dispositif de stérilisation selon au moins l'une des revendications 17 ou 18, **caractérisé en ce que** l'élément de positionnement (6) est susceptible d'être sollicité par la cloche stérile (8, 45) à sa position stérile.

20. Dispositif de stérilisation selon au moins l'une des revendications 17 à 19, **caractérisé en ce que** l'élément de positionnement (35) est disposé sur la cloche stérile (8, 45).

21. Dispositif de stérilisation selon la revendication 20, **caractérisé en ce que** l'élément de positionnement (35) sur la cloche stérile (8, 45) est réalisé sous la forme d'évidements (33), pour recevoir un tronçon de récipient.

22. Dispositif de stérilisation selon au moins l'une des revendications 17 à 21, **caractérisé en ce que** l'élément de positionnement (35, 6) est déplaçable par rapport au dispositif de remplissage (14).

23. Dispositif de stérilisation selon au moins l'une des revendications 17 à 22, **caractérisé en ce que** la tête de remplissage (13) est déplaçable par rapport à l'élément de positionnement (6, 35).

24. Dispositif de stérilisation selon au moins l'une des revendications 17 à 23, **caractérisé en ce qu'**à chaque élément de positionnement (6) est précisément associée une cloche stérile (8, 45).

25. Dispositif de stérilisation selon au moins l'une des revendications 17 à 24, **caractérisé en ce qu'**à chaque élément de positionnement (35) et/ou à chaque cloche stérile (8, 45) est précisément associée une tête de remplissage (13).

26. Dispositif de stérilisation selon au moins l'une des revendications 17 à 25, **caractérisé en ce que** l'élément de positionnement (6) et l'élément de fermeture sont identiques, **en ce que** le récipient (2) stérilisé est déplaçable au moyen d'un élément de positionnement (6), en une position stérile, à laquelle la cloche stérile (8, 45) entoure l'ouverture de remplissage (3) du récipient (2).

27. Dispositif de stérilisation selon au moins l'une des revendications précédentes, **caractérisé par** une commande à came (39), pour commander l'élément de fermeture (6, 38).

28. Dispositif de stérilisation selon au moins la revendication 27, **caractérisé en ce que** la commande à came (39) est prévue pour commander l'élément de fermeture, sur un dispositif de transport prévu pour les récipients (2) à stériliser.
